(19)

Europäisches
Patentamt
European
Patent Office
Office européen
des brevets

(11) **EP 4 447 054 A1**

(12) **EUROPEAN PATENT APPLICATION**

(43) Date of publication:
**16.10.2024   Bulletin 2024/42**

(21) Application number: **23205209.2**

(22) Date of filing: **23.10.2023**

(51) International Patent Classification (IPC):
**G16B 20/20** (2019.01)      **G16B 40/20** (2019.01)

(52) Cooperative Patent Classification (CPC):
**G16B 40/20; G16B 20/20**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **14.04.2023   KR 20230049291**

(71) Applicant: **3Billion
Seoul (KR)**

(72) Inventors:
• **LEE, Kyongyeul**
  **Seoul (KR)**
• **KIM, Ho Heon**
  **Seoul (KR)**
• **BAEK, Joo Yeup**
  **Seoul (KR)**

(74) Representative: **Frenkel, Matthias Alexander
Wuesthoff & Wuesthoff
Patentanwälte und Rechtsanwalt PartG mbB
Schweigerstraße 2
81541 München (DE)**

(54)  **SYSTEM AND METHOD FOR IDENTIFYING GENETIC DISEASE AND DISCOVERING DISEASE ASSOCIATED GENETIC VARIANTS BASED ON MULTIPLE INSTANCE LEARNING**

(57)    The present disclosure provides a system configured to identify a genetic disease and discover a disease-associated genetic variant, the system including a multiple instance learning model unit configured to derive identification of a genetic disease of a patient and discovery of a disease-associated genetic variant together using a multiple instance learning model configured to learn instances which are genetic variant information of the patient and a bag of the instances as input data and process, as a bag label, whether a disease of the patient is a genetic disease caused by a genetic variant.

EP 4 447 054 A1

**Description**

**CROSS-REFERENCE TO RELATED APPLICATION**

The present application is based upon and claims the benefit of priority to Korean Patent Application No. 10-2023-0049291 filed on 2023.04.14. The disclosures of the above-listed applications are hereby incorporated by reference herein in their entirety.

**BACKGROUND**

**1. Technical Field**

The present disclosure relates to a system for and a method of identifying a genetic disease and discovering disease-associated genetic variants such that whether a disease of a patient is a genetic disease may be determined using multiple instance learning and a disease-associated genetic variant, among several genetic variants in the patient, may be discovered.

**2. Description of Related Art**

A genetic disease is a disease caused by a genetic variant or a chromosomal aberration. A disease-associated genetic variant means a genetic variant that causes a genetic disease.
Interpretation of human genetic variants is a process of finding one or more disease-associated genetic variants among several genetic variants.
Generally, in order to identify a disease-associated genetic variation, a method of comparing genomic information of patients with a particular disease (cases) with genomic information of general healthy population (controls) to identify a genetic variant which is found significantly more in the cases than in the controls is being used.
Recently, research has been conducted into using artificial intelligence to determine whether a patient's disease is a genetic disease or to search for a disease-associated generic variant that causes the patient's disease.
There is research conducted to identify a disease-associated genetic variant for a patient's disease with respect to each of genetic variants in the patient using single instance learning. However, since such single instance learning needs a label for each of the genetic variants (instance label), use of the single instance learning is limited in reality.
The present disclosure presents a method of simultaneously determining whether a disease of a patient is a genetic disease, and a disease-causing genetic variant among several genetic variants using a multiple instance learning (MIL) model.

**SUMMARY**

The present disclosure provides a system for and a method of identifying a genetic disease and discovering disease-associated genetic variants such that whether a disease of a patient is a genetic disease, and a disease-associated genetic variant among several genetic variants in the patient are simultaneously determined using multiple instance learning.
To accomplish the above-mentioned objects, according to an aspect of the present disclosure, there is provided a system configured to identify a genetic disease and discover a disease-associated genetic variant, the system including a multiple instance learning model unit configured to derive identification of a genetic disease of a patient and discovery of a disease-associated genetic variant together using a multiple instance learning model configured to learn instances which are genetic variant information of the patient and a bag of the instances as input data and process, as a bag label, whether a disease of the patient is a genetic disease caused by a genetic variant.
The system may include an input data processing unit configured to generate attention weights which are degrees to which the instances contribute to the identification of a genetic disease of the patient using an attention mechanism, and process the input data by reflecting the attention weights for the instances.
The input data processing unit may include: a genetic variant information embedding unit configured to embed the respective instances into low-dimensional vectors with a same dimension using respective neural networks, and then, project the low-dimensional vectors onto one manifold using weight matrices and an activation function to obtain embedding vectors identical to each other; and a genetic variant information pooling unit configured to generate attention weights for the embedding vectors using the attention mechanism, and perform a pooling process of treating the embedding vectors as one vector.
The system may include a disease and associated genetic variant determination unit configured to determine that the disease of the patient is a genetic disease caused by a genetic variant when the embedding vectors are equal to or greater than a preset reference, and discover a disease-associated genetic variant that causes the disease of the patient using the attention weights for the instances.

The multiple instance learning model may be a multi-input model using input data with various vector magnitudes.

An instance label for the instances may be generated using the attention weights for the instances, and the multiple instance learning model may be retrained using the instance label.

According to another aspect of the present disclosure, there is provided a method of identifying a genetic disease and discovering a disease-associated genetic variant, the method including: processing input data such that an input data processing unit uses instances which are genetic variant information of a patient and a bag of the instances as input data, and generates attention weights for the instances using an attention mechanism to process the input data; identifying presence of a genetic disease such that a multiple instance learning model unit identifies whether a disease of the patient is a genetic disease using a multiple instance learning model; and discovering a disease-associated generic variant such that when the disease of the patient is determined to be a genetic disease, a disease and associated genetic variant determination unit discovers a disease-associated genetic variant that causes the disease of the patient using the attention weights for the instances.

The method may further include retraining such that, when the disease of the patient is determined to be a genetic disease, an instance label is generated using the attention weights for the instances, and the multiple instance learning model is retrained using the generated instance label.

## BRIEF DESCRIPTION OF THE DRAWINGS

FIG. 1 is a configuration diagram of a system configured to identify a genetic disease and discover a disease-associated genetic variant, input data, and output data according to an embodiment of the present disclosure.

FIG. 2 is a configuration diagram of the system configured to identify a genetic disease and discover a disease-associated genetic variant, input data, and output data according to an embodiment of the present disclosure.

FIG. 3 is a configuration diagram of the system configured to identify a genetic disease and discover a disease-associated genetic variant according to an embodiment of the present disclosure.

FIG. 4 is a diagram illustrating an execution process performed by the system configured to identify a genetic disease and discover a disease-associated genetic variant according to an embodiment of the present disclosure.

FIG. 5 is a diagram illustrating a result of a test on discovering a disease-associated genetic variant using the system configured to identify a genetic disease and discover a disease-associated genetic variant according to an embodiment of the present disclosure.

FIG. 6 is a flowchart of a method of identifying a genetic disease and discovering a disease-associated genetic variant according to an embodiment of the present disclosure.

## DETAILED DESCRIPTION

It will be understood that terms such as "include" or "have," when used herein, are not intended to preclude a possibility that one or more other features, numbers, steps, operations, components, parts, or combinations thereof may exist or may be added.

In this specification, the singular includes the plural unless specifically stated otherwise. For example, an instance may be described in singular, but a plurality of instances may be also included.

Hereinafter, to obviate those problems, an embodiment of the present disclosure will be described in detail with reference to the accompanying drawings.

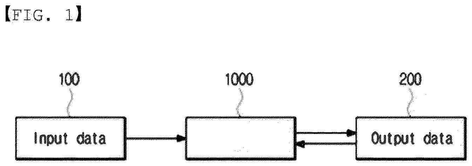

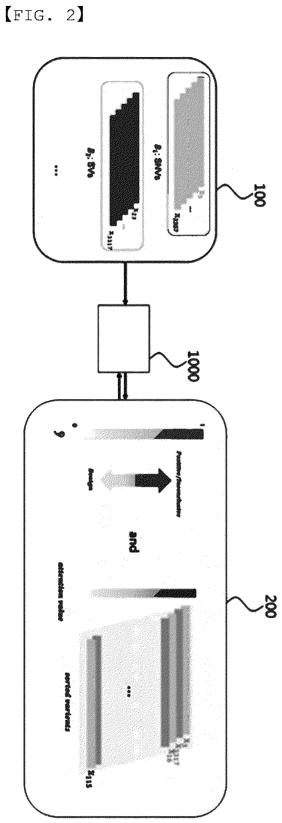

FIGS. 1 and 2 are configuration diagrams of a system 1000 configured to identify a genetic disease and discover a disease-associated genetic variant, input data, and output data according to an embodiment of the present disclosure.

Referring to FIGS. 1 and 2, the system 1000 configured to identify a genetic disease and discover a disease-associated genetic variant according to an embodiment of the present disclosure may learn input data 100 to generate output data 200.

The system 1000 configured to identify a genetic disease and discover a disease-associated genetic variant according to an embodiment of the present disclosure may use a multiple instance learning model and an attention mechanism.

The multiple instance learning model presents a type of supervised learning and provides a learning method of, when several data points (=instances) are constituted as one bundle (bag), handling a classification of the bundle (bag).

The system 1000 configured to identify a genetic disease and discover a disease-associated genetic variant according to the present disclosure handles information of each of genetic variants as an instance and learns a bag of instances as input data 100, and whether a disease of a patient is a genetic disease caused by a genetic variant is a bag label, and the bag label is handled as output data 200. In this case, the multiple instance learning model may be a multi-input model using input data with various vector magnitudes.

As an example, the input data 100 may have a genetic variant in the patient as an instance, and include a bundle (bag) of various genetic variants (e.g., a single nucleotide variant (SNV), a structural variant (SV), a copy number variant (CNV), etc.).

A single nucleotide variant (SNV) is a single base mutation, and means a substitution of one base for another base in a deoxyribonucleic acid (DNA) base sequence. For example, when C is changed to T, this is known as a C-to-T mutation or single nucleotide polymorphism (SNP).

A structural variant (SV) refers to a large structural alteration within a gene. This structural alteration usually occurs when DNA base sequences in two regions are moved, deleted, duplicated, or reversed. This structural alteration may have a great effect on the DNA base sequence.

A copy number variant (CNV) refers to a case in which a particular DNA base sequence is present in two or more copies. The CNV is also known as a genetic cause associated with a human disease.

The system 1000 configured to identify a genetic disease and discover a disease-associated genetic variant according to the present disclosure may determine whether the disease of the patient is a genetic disease caused by a genetic variant using the multiple instance learning model, and discover a disease-associated genetic variant using the attention mechanism. That is, the output data 200 may be a result of identifying whether the disease of the patient is a genetic disease caused by a genetic variant, and a disease-associated genetic variant among several genetic variants in the patient.

As an example, whether the disease of the patient is a genetic disease caused by a genetic variant ($\hat{y}$) may be expressed as a value from 0 to 1, and when the value is equal to or greater than a preset reference value, the disease of the patient may be identified as a genetic disease caused by a genetic variant.

Also, as will be described later, a disease-associated genetic variant may be identified among several genetic variants in the patient using attention weights for respective genetic variants generated using the attention mechanism.

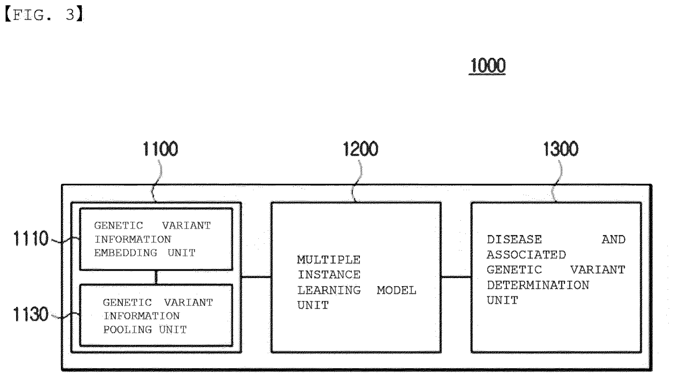

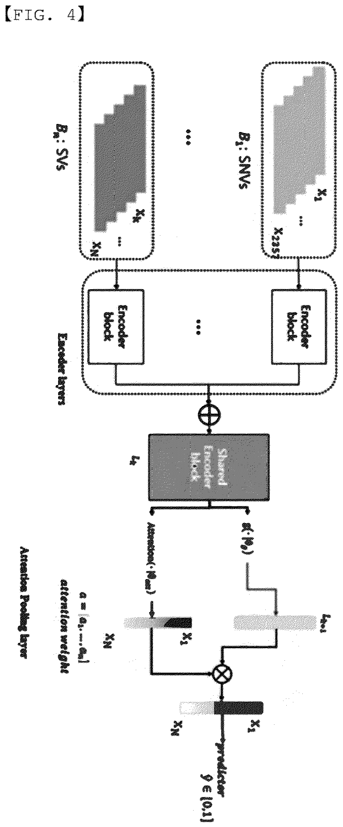

FIG. 3 is a configuration diagram of the system configured to identify a genetic disease and discover a disease-associated genetic variant according to an embodiment of the present disclosure. FIG. 4 is a diagram illustrating an execution process performed by the system configured to identify a genetic disease and discover a disease-associated genetic variant according to an embodiment of the present disclosure.

Referring to FIGS. 3 and 4, the system 1000 configured to identify a genetic disease and discover a disease-associated genetic variant according to an embodiment of the present disclosure includes an input data processing unit 1100, a multiple instance learning model unit 1200, and a disease and associated genetic variant determination unit 1300.

The input data processing unit 1100 may use an attention mechanism to generate an attention weight, which is a degree to which each of the instances contributes to identification of a genetic disease of the patient, and process input data by reflecting the attention weight for each of the instances.

The attention mechanism provides a processing method capable of performing learning and identification by focusing on important parts of the input data in a deep learning model. In a case of general deep learning, all parts of input data are processed with equal weight. Thus, even when a part of the input data contains important information, it was difficult to recognize important information. The attention mechanism is used to help the deep learning model to learn to find parts with importance in the input data and perform calculation by multiplying an input by a degree of the importance to allow the deep learning model to improve recognition of important information.

The input data processing unit 1100 includes a genetic variant information embedding unit 1110 and a genetic variant information pooling unit 1130.

The genetic variant information embedding unit 1110 may embed the respective instances into low-dimensional vectors with a same dimension using each neural network, and then, project the low-dimensional vectors onto one manifold using weight matrices and an activation function to obtain same embedding vectors.

The genetic variant information pooling unit 1130 may generate attention weights for embedding vectors using the attention mechanism, and perform a pooling process of treating the embedding vectors as one vector.

For example, when two types of mutations such as an SNV and an SV are transmitted as input values, a set (bag) of variants may be defined with an expression as shown in Equation 1, and an instance (($x_i$, $x_i$)) may be defined with an expression as shown in Equation 2. Additionally, the SNV has f feature values, and the SV has h feature values.

Equation 1

$$B = \{B_{snv}, B_{sv}\}$$

Equation 2

$$B_{snv} = \{x_1, \ldots, x_{m_1}\}$$

$$B_{sv} = \{x_1', \ldots, x_{m_2}'\}$$

where $x_i \in \mathbb{R}^J$ and $x_i' \in \mathbb{R}^A$

As shown in Equations 3 and 4 below, the genetic variant information embedding unit 1110 may embed the SNV and the SV in the patient into low-dimensional vectors with a same dimension using respective neural networks, and then, project the low-dimensional vectors onto one manifold using a same weight matrix.

Equation 3

$$L_{k,snv} = f_{snv}(B_{snv})$$

$$L_{k,sv} = f_{sv}(B_{sv})$$

Equation 4

$$\text{for } L_k = L_{k,snv} \oplus L_{k,sv}$$

$$L_{k+1} = g(L_k | \theta_g)$$

where $L_{k+1} \in \mathbb{R}^{n \times f}$ ; $\theta_g$ is trainable parameters, $g(\cdot)$ embedding layer with non-linear activation function.

Here, a concatenation of a matrix or vector is denoted by $\oplus$, and an element wise product of the matrix is denoted by $\odot$.

The genetic variant information pooling unit 1130 may obtain attention weights for embedding vectors obtained for respective genetic variants and obtain a weighted sum of the respective embedding vectors. The obtained attention weights may be regarded as importance degrees of the respective variants, and a variant having a large value of an importance degree may be interpreted as a disease-associated generic variant.

The genetic variant information pooling unit 1130 may obtain attention weights for individual genetic variants using the attention mechanism to obtain importance degrees of embedding vectors obtained for the individual genetic variants. After passing the embedding vectors through a two-layer neural network as shown in Equation 5, the attention weights may be obtained by passing the embedding vectors through a softmax function as shown in Equation 6.

Equation 5

$$\text{for } L_{k+1} = [l_1, \ldots, l_n]^T \in R^{n \times f}, \; l_i \text{ is a row vector for } L_{k+1}.$$

$$l_i^+ = tanh(l_i W_1) w_2$$

where $W_1 \in \mathbb{R}^{f \times (f/4)}, w_2 \in \mathbb{R}^{(f/4) \times 1}$

Equation 6

$$a_i = \frac{e^{l_i^+}}{\sum_j e^{l_j^+}}$$

where e is a natural constant.

As shown in Equation 7 below, an aggregated embedding vector for a disparity axis may be obtained by calculating a dot product of respective column vectors and the obtained attention weights in a matrix with the embedding vectors obtained in Equation 4 as row vectors.

Equation 7

$$for\ a = [a_1, \dots, a_n]$$

$$z = aL_{k+1}$$

where z is $f$ - dimensional vector

The multiple instance learning model unit 1200 may predict a degree of a possibility in which the patient may actually have a genetic disease, by passing the aggregated embedding vectors through a single-layer neural network, as shown in Equation 8 below.

Equation 8

$$\hat{y} = \sigma(zW_z + b_z)$$

where $\sigma(\cdot)$ is sigmoid function,

and $W_z \in \mathbb{R}^{f \times 1}, b_z \in \mathbb{R}^1$ are trainable parameters.

The disease and associated genetic variant determination unit 1300 may determine that the disease of the patient is a genetic disease caused by a genetic variant when the aggregated embedding vectors are equal to or greater than a preset reference, and thus, discover a disease-associated genetic variant that causes the disease of the patient by using the attention weights for the instances.

The disease and associated genetic variant determination unit 1300 may order genetic variants according to contribution values obtained by calculating a dot product of the attention weights obtained in Equation 6 and encoded genetic variant information. Then, preset high-rank genetic variants may be regarded as disease-associated generic variants.

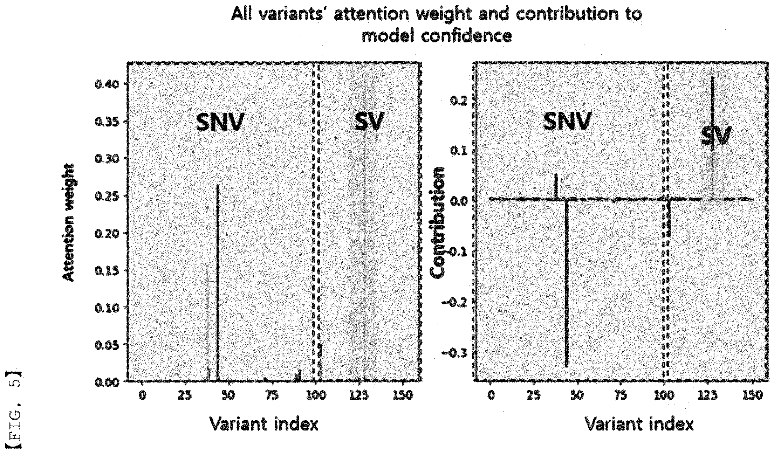

FIG. 5 is a diagram illustrating a result of a test on discovering a disease-associated genetic variant using the system configured to identify a genetic disease and discover a disease-associated genetic variant according to an embodiment of the present disclosure.

FIG. 5 illustrates identification of a genetic disease of a patient with a total of 157 variants including 100 SNVs and 57 SVs using the system configured to identify a genetic disease and discover a disease-associated genetic variant according to the present disclosure, and visualization of contributions according to each variant.

In FIG. 5, points 0 to 99 in an x-axis represent SNV indices and points 101 to 157 represent SV indices. Whether a genetic disease is present is identified with a model confidence of 0.6, and it may be interpreted that a 128th variant among the SNVs and SVs contributes most. Since the system configured to identify a genetic disease and discover a disease-associated genetic variant according to the present disclosure obtains a value of 0.6 by focusing on the 128th variant with respect to a disease-associated generic variant, the 128th variant may be interpreted as a disease-associated genetic variant.

As such, the system configured to identify a genetic disease and discover a disease-associated genetic variant according to the present disclosure may determine whether a disease is a genetic disease, using the multi instance learning model without having to use a genetic variant label (instance label) of a patient.

In addition, a disease-associated generic variant in a patient may be discovered using attention weights for the patient's genetic variants generated using the attention mechanism.

That is, the system 1000 configured to identify a genetic disease and discover a disease-associated genetic variant according to the present disclosure may simultaneously determine whether a patient has a genetic disease, and a disease-associated genetic variant using the attention mechanism and the multiple instance learning model without having to use a genetic variant label (instance label) of the patient.

In addition, the system 1000 configured to identify a genetic disease and discover a disease-associated genetic variant according to the present disclosure may improve performance of the multiple instance learning model by generating a genetic variant label (instance label) for a patient using an obtained attention weight and by retraining the multiple instance learning model using the generated instance label.

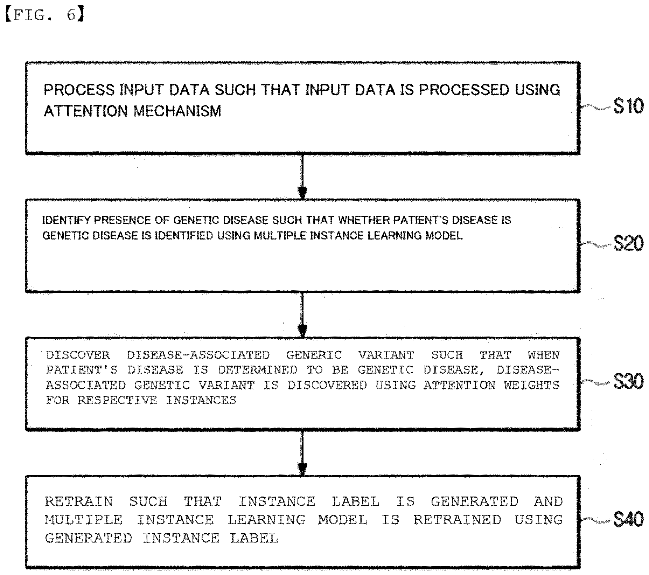

FIG. 6 is a flowchart of a method of identifying a genetic disease and discovering a disease-associated genetic variant according to an embodiment of the present disclosure.

The method of identifying a genetic disease and discovering a disease-associated genetic variant according to an embodiment of the present disclosure includes input data processing (S10), identifying presence of a genetic disease (S20), and discovering a disease-associated generic variant (S30) . and a retraining (S40) .

In the input data processing (S10), an input data processing unit uses instances, i.e., genetic variant information of a patient and a bag of the instances as input data, and generate attention weights for the instances using an attention mechanism to process the input data.

In the identifying of presence of a genetic disease (S20), a multiple instance learning model unit may identify whether a disease of the patient is a genetic disease using a multiple instance learning model.

In the discovering a disease-associated generic variant (S30), when the patient's disease is determined to be a genetic disease, a disease and associated genetic variant determination unit may discover a disease-associated genetic variant which causes the patient's disease using the attention weights for the instances.

In this case, when the patient's disease is not determined to be a genetic disease, the discovering of a disease-associated generic variant may not be performed.

In the retraining (S40), when the patient's disease is determined to be a genetic disease, an instance label may be generated using the attention weights for the instances, and the multiple instance learning model is retrained using the generated instance label. According to the method of identifying a genetic disease and discovering a disease-associated genetic variant according to an embodiment of the present disclosure, the multiple instance learning model may be retrained to improve performance of the multiple instance learning model.

Although the illustrative configuration of an apparatus has been shown in the present specification and the attached drawings, functional operations and implementations of the subject matter described herein may be implemented in different types of digital electronic circuits, or may be implemented in the form of computer software, firmware, or hardware including the structures disclosed herein and structural equivalents thereof or may be implemented by a combination of one or more thereof. Implementations of the subject matter described herein may include one or more computer program products, i.e., one or more modules regarding computer program instructions encoded on a tangible program storage medium to control operations of an apparatus according to the present disclosure or perform execution based on the operations. The computer-readable medium may be a machine-readable storage device, a machine-readable storage substrate, a memory device, or a combination thereof.

The present disclosure described above is not limited to the embodiments set forth herein, and it should be apparent to those skilled in the art the accompanying drawings, and various substitutions, modifications and changes may be made therein without departing from the spirit and scope of the present disclosure.

**ADVANTAGEOUS EFFECTS**

The present disclosure may determine whether a patient has a genetic disease without having to have a genetic variant label (instance label) using a multiple instance learning model.

In addition, in the present disclosure, a disease-associated generic variant in a patient may be discovered using attention weights for the patient's genetic variants generated using an attention mechanism.

In addition, in the present disclosure, whether a patient has a genetic disease and a disease-associated genetic variant may be simultaneously determined using the attention mechanism and a multiple instance learning model without having to have a genetic variant label (instance label) of the patient.

In addition, in the present disclosure, performance of the multiple instance learning model may be improved by generating the patient's genetic variant label (instance label) using the attention weights and retraining the multiple instance learning model using the generated instance label.

**Claims**

1. A system configured to identify a genetic disease and discover a disease-associated genetic variant, the system comprising a multiple instance learning model unit configured to derive identification of a genetic disease of a patient and discovery of a disease-associated genetic variant together using a multiple instance learning model configured to learn instances which are genetic variant information of the patient and a bag of the instances as input data and process, as a bag label, whether a disease of the patient is a genetic disease caused by a genetic variant.

2. The system of claim 1, comprising an input data processing unit configured to generate attention weights which are degrees to which the instances contribute to the identification of a genetic disease of the patient using an attention mechanism, and process the input data by reflecting the attention weights for the instances.

3. The system of claim 2, wherein the input data processing unit comprises:

   a genetic variant information embedding unit configured to embed the respective instances into low-dimensional vectors with a same dimension using respective neural networks, and then, project the low-dimensional vectors onto one manifold using weight matrices and an activation function to obtain embedding vectors identical to each other; and
   a genetic variant information pooling unit configured to generate attention weights for the embedding vectors using the attention mechanism, and perform a pooling process of treating the embedding vectors as one vector.

4. The system of claim 3, comprising a disease and associated genetic variant determination unit configured to determine that the disease of the patient is a genetic disease caused by a genetic variant when the embedding vectors are equal to or greater than a preset reference, and discover a disease-associated genetic variant that causes the disease of the patient using the attention weights for the instances.

5. The system of any one of claims 1 to 4, wherein the multiple instance learning model is a multi-input model using input data with various vector magnitudes.

6. The system of claim 4, wherein an instance label for the instances is generated using the attention weights for the instances, and the multiple instance learning model is retrained using the instance label.

7. A method of identifying a genetic disease and discovering a disease-associated genetic variant, the method comprising:

   processing input data such that an input data processing unit uses instances which are genetic variant information of a patient and a bag of the instances as input data, and generates attention weights for the instances using an attention mechanism to process the input data;
   identifying presence of a genetic disease such that a multiple instance learning model unit identifies whether a disease of the patient is a genetic disease using a multiple instance learning model; and
   discovering a disease-associated generic variant such that when the disease of the patient is determined to be a genetic disease, a disease and associated genetic variant determination unit discovers a disease-associated genetic variant that causes the disease of the patient using the attention weights for the instances.

8. The method of claim 7, further comprising retraining such that, when the disease of the patient is determined to be a genetic disease, an instance label is generated using the attention weights for the instances, and the multiple instance learning model is retrained using the generated instance label.

【FIG. 1】

100                          1000                          200

| Input data |  →  |        |  ⇄  | Output data |

【FIG. 2】

【FIG. 3】

1000

1100                    1200                    1300

| 1110 | GENETIC VARIANT INFORMATION EMBEDDING UNIT |
| 1130 | GENETIC VARIANT INFORMATION POOLING UNIT |

MULTIPLE INSTANCE LEARNING MODEL UNIT

DISEASE AND ASSOCIATED GENETIC VARIANT DETERMINATION UNIT

【FIG. 4】

【FIG. 5】

All variants' attention weight and contribution to model confidence

【FIG. 6】

PROCESS INPUT DATA SUCH THAT INPUT DATA IS PROCESSED USING ATTENTION MECHANISM ~S10

IDENTIFY PRESENCE OF GENETIC DISEASE SUCH THAT WHETHER PATIENT'S DISEASE IS GENETIC DISEASE IS IDENTIFIED USING MULTIPLE INSTANCE LEARNING MODEL ~S20

DISCOVER DISEASE-ASSOCIATED GENERIC VARIANT SUCH THAT WHEN PATIENT'S DISEASE IS DETERMINED TO BE GENETIC DISEASE, DISEASE-ASSOCIATED GENETIC VARIANT IS DISCOVERED USING ATTENTION WEIGHTS FOR RESPECTIVE INSTANCES ~S30

RETRAIN SUCH THAT INSTANCE LABEL IS GENERATED AND MULTIPLE INSTANCE LEARNING MODEL IS RETRAINED USING GENERATED INSTANCE LABEL ~S40

Europäisches
Patentamt

European
Patent Office

Office européen
des brevets

# EUROPEAN SEARCH REPORT

Application Number

EP 23 20 5209

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NIEBOER MARLEEN M. ET AL: "Predicting pathogenic non-coding SVs disrupting the 3D genome in 1646 whole cancer genomes using multiple instance learning", SCIENTIFIC REPORTS, [Online] vol. 11, no. 1, 13 July 2021 (2021-07-13), XP093144562, US ISSN: 2045-2322, DOI: 10.1038/s41598-021-93917-y Retrieved from the Internet: URL:https://www.ncbi.nlm.nih.gov/pmc/artic les/PMC8277903/pdf/41598_2021_Article_9391 7.pdf> [retrieved on 2024-03-22] | 1 | INV. G16B20/20 G16B40/20 |
| Y | * Abstract; Introduction (Par. 6); Results (section "svMIL2 can accurately predict driver genes disrupted by non-coding SVs across cancer types"); Methods (sections "Data", "svMIL2 model"); figure 1 * | 2-8 | |
| | ----- | | |
| X | CN 111 489 788 A (UNIV BEIHANG) 4 August 2020 (2020-08-04) | 1 | TECHNICAL FIELDS SEARCHED (IPC) |
| Y | * the whole document * | 2-8 | G16B |
| | ----- | | |
| | -/-- | | |

The present search report has been drawn up for all claims

| Place of search | Date of completion of the search | Examiner |
|---|---|---|
| Munich | 25 March 2024 | Hendrikse, Natalie |

EPO FORM 1503 03.82 (P04C01)

Europäisches
Patentamt
European
Patent Office
Office européen
des brevets

# EUROPEAN SEARCH REPORT

Application Number

EP 23 20 5209

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | YIXIN CHEN ET AL: "MILES: Multiple-Instance Learning via Embedded Instance Selection", IEEE TRANSACTIONS ON PATTERN ANALYSIS AND MACHINE INTELLIGENCE, IEEE COMPUTER SOCIETY, USA, vol. 28, no. 12, 12 October 2006 (2006-10-12), pages 1931-1947, XP001523410, ISSN: 0162-8828, DOI: 10.1109/TPAMI.2006.248 | 2-8 | |
| A | * Abstract; Sections 1.1, 1.3, 2.2, and 5.3.3 * | 1 | |
| | ----- | | |
| X | US 2021/117815 A1 (CREED PAIDI [GB] ET AL) 22 April 2021 (2021-04-22) | 1 | |
| Y | * paragraphs [0012] - [0016], [0096], [0213]; figures 1a-1d, 2a-2d * | 2-8 | |
| | ----- | | |

TECHNICAL FIELDS
SEARCHED (IPC)

The present search report has been drawn up for all claims

| Place of search | Date of completion of the search | Examiner |
|---|---|---|
| Munich | 25 March 2024 | Hendrikse, Natalie |

EPO FORM 1503 03.82 (P04C01)

## ANNEX TO THE EUROPEAN SEARCH REPORT
## ON EUROPEAN PATENT APPLICATION NO.

EP 23 20 5209

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2024

| Patent document cited in search report | | Publication date | Patent family member(s) | | Publication date |
|---|---|---|---|---|---|
| CN 111489788 | A | 04-08-2020 | NONE | | |
| US 2021117815 | A1 | 22-04-2021 | CN | 112136145 A | 25-12-2020 |
| | | | EP | 3776379 A1 | 17-02-2021 |
| | | | US | 2021117815 A1 | 22-04-2021 |
| | | | WO | 2019186198 A1 | 03-10-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230049291